# EUROPÄISCHE PATENTSCHRIFT

(11) **EP 3 458 008 B1**
(45) Veröffentlichungstag und Bekanntmachung des Hinweises auf die Patenterteilung: **28.06.2023**
(21) Anmeldenummer: 17728042.7
(22) Anmeldetag: 18.05.2017
(51) Int. Cl.: A61G 5/10, A61G 5/12

(54) **EINRICHTUNG ZUR BEFESTIGUNG EINER ARMSTÜTZSTRUKTUR**
DEVICE FOR SECURING AN ARM SUPPORT STRUCTURE
DISPOSITIF POUR FIXER UNE STRUCTURE DE SUPPORT D'UN BRAS

(30) Priorität: 18.05.2016 AT 504602016
(43) Veröffentlichungstag der Anmeldung: 27.03.2019
(73) Patentinhaber: Technische Universität Wien, 1040 Wien (AT)
(72) Erfinder: PUCHINGER, Markus, 3100 St. Pölten (AT)
(74) Vertreter: SONN Patentanwälte OG
(86) Internationale Anmeldenummer: PCT/AT2017/060132
(87) Internationale Veröffentlichungsnummer: WO 2017/197424

(56) Entgegenhaltungen:
- EP-A1- 2 957 392
- WO-A1-2016/166652
- WO-A2-2015/099858
- TARIQ RAHMAN ET AL: "Passive exoskeletons for assisting limb movement", THE JOURNAL OF REHABILITATION RESEARCH AND DEVELOPMENT, Bd. 43, Nr. 5, 30. September 2006 (2006-09-30), Seite 583, XP055193282, ISSN: 0748-7711, DOI: 10.1682/JRRD.2005.04.0070
- Peter H Stern ET AL: "Wheelchair Based Upper Limb Orthotics Shoulder-Arm Modules", , 31. Dezember 1982 (1982-12-31), Seiten 41-45, XP055395566, Gefunden im Internet: URL:http://www.oandplibrary.org/op/pdf/198 2_01_041.pdf [gefunden am 2017-08-02]

## Beschreibung

Die Erfindung betrifft eine Einrichtung zur Befestigung einer Arm-Stützstruktur zur Unterstützung der Bewegung eines Armes, beispielsweise einer Orthese oder eines Arm-Exoskeletts, an einem Rollstuhl oder dgl., mit einem Verbindungselement zur Verbindung mit der Arm-Stützstruktur, und mit einem Verbindungselement zur Verbindung mit dem Rollstuhl oder dgl..

Arm-Stützstrukturen sind beispielsweise in Form von Orthesen oder Arm-Exoskeletten bekannt und dienen zur Schaffung einer stabilen Hülle um den Arm bzw. die Extremität. Eine Orthese dient zur Stabilisierung, Entlastung, Ruhigstellung oder Führung einer Gliedmaße oder des Rumpfes und werden beispielsweise nach Verletzungen eingesetzt. Künstliche Exoskelette für die untere und obere Extremität werden zur Unterstützung und Verstärkung der Bewegung des Trägers und zur Rehabilitation und Verbesserung der Lebensqualität von Personen mit Einschränkungen der motorischen Fähigkeiten angewendet.

Beispielsweise beschreibt die WO 2015/084177 A1 ein Arm-Exoskelett, welches vom Patienten am Körper getragen wird. Somit muss das gesamte Gewicht des Arm-Exoskeletts vom Träger getragen werden, was eine Durchführung von Bewegungen des jeweiligen Armes erschwert.

Die EP 2 957 392 A1 beschreibt eine Vorrichtung zur motorunterstützten Bewegung einer menschlichen Gliedmaße über ein Exoskelett, welche zur Vermeidung einer falschen Ausrichtung des Exoskeletts in Bezug auf die biologischen Gelenke des Patienten sowie zur Vermeidung einer kardanischen Blockade entsprechend ausgebildet ist. Die Bewegung zweier Antriebe wird über eine Konstruktion bestehend aus zwei Schubstangen und einer Drehstange auf das Exoskelett übertragen.

Abhilfe dagegen schaffen Arm-Exoskelette welche an einem Stuhl, Rollstuhl oder dgl. befestigt werden, wodurch der Großteil des Gewichts des Arm-Exoskeletts über den Stuhl, Rollstuhl oder dgl. abgestützt werden kann. Auch ist mit solchen Einrichtungen eine Behandlung von Personen mit eingeschränkter Mobilität, welche im Rollstuhl sitzen müssen, möglich. Beispielsweise beschreibt die US 2007/0255190 A1 ein solches, am Rollstuhl befestigtes Exoskelett.

Üblicherweise ist die Verbindung zwischen Arm-Stützstruktur bzw. Arm-Exoskelett und Rollstuhl starr mit Verbindungsstangen oder dgl. ausgestaltet, wodurch die Bewegungsfreiheit des Patienten im Bereich des Oberkörpers stark eingeschränkt und insbesondere in den Pausen zwischen den Bewegungsübungen als belastend empfunden wird.

Die Veröffentlichung von Tariq Rahman et al: "Passive exoskeletons for assisting limb movement " (The Journal of Rehabilitation Research and Development, Bd. 43, Nr. 5, 30. September 2006, Seite 583, XP055193282, ISSN: 0748-7711, DOI: 10.1682/JRRD.2005.04.0070) beschreibt ein Arm-Exoskelett, das durch eine gebogene Stange starr an einem Rollstuhl befestigt ist und somit keine Bewegung des Oberkörpers des Benutzers bei angelegter Stützstruktur ermöglicht.

Die Aufgabe der vorliegenden Erfindung besteht in der Schaffung einer oben genannten Einrichtung, durch welche das Gewicht der Arm-Stützstruktur, beispielsweise am Rollstuhl oder dgl. abgestützt werden kann, aber dennoch zumindest zeitweise eine gewisse Bewegungsfreiheit im Bereich des Oberkörpers des Patienten ermöglicht wird. Nachteile bekannter Einrichtungen sollen verhindert oder zumindest reduziert werden.

Gelöst wird die erfindungsgemäße Aufgabe dadurch, dass das Verbindungselement zur Verbindung mit der Arm-Stützstruktur durch eine in der Gebrauchslage im Wesentlichen vertikal angeordnete, um einen Winkel um ihre Längsachse drehbare Verbindungsstange gebildet ist, und das Verbindungselement zur Verbindung mit dem Rollstuhl oder dgl. durch eine in der Gebrauchslage im Wesentlichen vertikal angeordnete, drehbare Verbindungsstange gebildet ist, und die Verbindungsstangen durch zwei parallele Querstangen über Drehgelenke beweglich miteinander verbunden sind, wobei die Verbindungsstangen und die Querstangen ein verstellbares Parallelogramm bilden, sodass die mit der Arm-Stützstruktur verbindbare Verbindungsstange sowohl in der Höhe, als auch im Winkel gegenüber der Verbindungsstange zur Verbindung mit dem Rollstuhl oder dgl. verstellbar ist. Durch diese Konstruktion wird gewährleistet, dass die Arm-Stützstruktur immer in der gewünschten Lage verbleibt, aber dennoch eine Neigung und Drehung des Oberkörpers während der Bewegungen mit der Arm-Stützstruktur oder zumindest in den Pausen zwischen den Bewegungen möglich ist. Durch das verstellbare Parallelogramm der Befestigungseinrichtung wird gewährleistet, dass die horizontale Achse im Schultergelenk des Patienten immer in der horizontalen Lage bleibt und eine Drehung um die vertikal angeordnete, drehbare Verbindungsstange möglich ist. Die Konstruktion ist relativ einfach aufgebaut und kann daher kostengünstig und mit geringem Gewicht hergestellt werden. Durch die drehbare Anordnung der mit der Arm-Stützstruktur verbindbaren Verbindungsstange wird ein weiterer Freiheitsgrad erzielt und somit der Tragekomfort für den Patienten noch weiter erhöht. Durch die Drehung dieser Verbindungsstange wird die Rotation in der Schulterelevationsebene ermöglicht.

Wenn die mit dem Rollstuhl oder dgl. verbindbare Verbindungsstange in Längsrichtung verschiebbar angeordnet ist, kann ein noch höherer Komfort für den Patienten erzielt werden. Die Verschiebbarkeit in Längsrichtung der Verbindungsstange kann durch eine teleskopartige Konstruktion gebildet werden.

Vorzugsweise ist eine Einrichtung zur Dämpfung der Verschiebung der Verbindungsstange in Längsrichtung vorgesehen. Dadurch wird eine gedämpfte Bewegung der Befestigungseinrichtung in der im Wesentlichen vertikalen Richtung erzielt. Die Dämpfungseinrichtung kann beispielsweise aus Moosgummi hergestellt sein, der an geeigneten Stellen und in geeigneter Weise auf die verschiebliche Verbindungsstange aufgebracht wird.

Die Verbindungsstangen und bzw. oder die Querstangen sind vorzugsweise aus faserverstärktem Kunststoff, insbesondere aus pultrudiertem Carbon-Faser-Kunststoff, gebildet. Diese Materialien zeichnen sich durch niedriges Gewicht und hohe Festigkeit aus.

Weiters können die Verbindungsstangen und bzw. oder die Querstangen hohl und zur Führung von Leitungen oder dgl. ausgebildet sein. Durch die hohle Ausbildung der Verbindungsstangen und bzw. oder der Querstangen kann das Gesamtgewicht der Befestigungseinrichtung noch weiter verringert werden und es können zusätzlich die hohl ausgebildeten Verbindungsstangen und bzw. oder Querstangen zur Führung von vorhandenen Leitungen oder dgl. verwendet werden. Beispielsweise können die Leitungen zur Versorgung eines motorisierten Arm-Exoskeletts mit elektrischer Energie über die hohl ausgebildeten Verbindungsstangen oder Querstangen verlegt werden, aber auch elektrische Leitungen von Sensoren durch die hohlen Verbindungsstangen oder Querstangen der Befestigungseinrichtung angeordnet werden. Auch allfällige Bowdenzüge können im Inneren der Verbindungsstangen oder Querstangen der Befestigungseinrichtung verlegt werden.

Wenn zwischen den Querstangen des Parallelogramms ein Federelement angeordnet ist, kann eine automatische Rückstellung des Parallelogramms in die Ausgangslage erzielt werden. Dadurch kann der Patient in die Ausgangslage gezwungen bzw. geführt werden und er kann durch Überwindung der Federkraft des Federelements diese Ausgangslage leicht verlassen.

Dabei kann das Federelement durch zumindest ein elastisches Band gebildet sein. Dies stellt eine besonders einfache und kostengünstige Ausführungsvariante dar. Dabei ist das zumindest eine elastische Band vorzugsweise zwischen den Querstangen des Parallelogramms der Befestigungseinrichtung angeordnet.

Alternativ dazu kann das Federelement auch durch zumindest eine Gasdruckfeder gebildet sein. Anstelle einer Gasdruckfeder kann natürlich auch eine mechanische Zugfeder zwischen den Querstangen des Parallelogramms der Befestigungseinrichtung angeordnet werden.

Zwischen den Querstangen des Parallelogramms kann ein Sperrelement zum Sperren der Verstellbarkeit des Parallelogramms vorgesehen sein oder das Federelement sperrbar ausgebildet sein. Da es für bestimmte Bewegungsabläufe notwendig oder sinnvoll sein kann, dass der Patient eine aufrecht sitzende Position einnimmt, kann es von Vorteil sein, die erfindungsgemäß beweglich ausgebildete Befestigungseinrichtung zumindest während dieser Bewegungen zu sperren bzw. zu blockieren.

Das Sperrelement kann beispielsweise durch eine diagonal zwischen den Querstangen angeordnete Stange gebildet sein. Dies stellt eine einfache mechanische Variante der Ausbildung eines Sperrelements dar. Natürlich können auch elektrische oder elektromechanische Sperrmechanismen, beispielsweise in den Gelenken des Parallelogramms, vorgesehen werden.

Die Arm-Stützstruktur kann durch ein Arm-Exoskelett mit einem Schultergelenksmodul gebildet sein, und die Verbindungsstange zur Verbindung mit der Arm-Stützstruktur zumindest teilweise durch das Schultergelenksmodul des Arm-Exoskeletts gebildet sein. Dadurch, dass ein Teil des Arm-Exoskeletts einen Teil der Befestigungseinrichtung bildet, kann das Gesamtgewicht der Arm-Stützstruktur zusammen mit der Befestigungseinrichtung weiter verringert werden.

Wenn das Schultergelenksmodul mit einer Gewichtskompensations-Einrichtung verbunden ist, kann das Gewicht des Arm-Exoskeletts kompensiert und das benötigte Moment reduziert werden.

Die Gewichtskompensations-Einrichtung kann gemäß einem weiteren Merkmal der Erfindung durch zumindest eine Feder gebildet sein, welche über einen innerhalb der Querstangen verlaufenden Bowdenzug verbunden ist. Dies stellt eine einfache und robuste Realisierungsmöglichkeit einer Gewichtskompensations-Einrichtung dar.

Wenn zumindest eine Feder zuschaltbar ausgebildet ist, kann eine Anpassung an das zu kompensierende Gewicht erzielt werden.

In zumindest einem Gelenk des Parallelogramms kann ein Winkelencoder angeordnet sein, um die Verstellung des Parallelogramms erfassen zu können. Dabei können die elektrischen Leitungen zu den Winkelencodern in hohl ausgebildeten Verbindungsstangen und Querstangen des Parallelogramms verlegt werden. Die Daten der Winkelencoder werden in entsprechenden Einrichtungen, beispielsweise Computern, erfasst und weiterverarbeitet oder zur Dokumentation gespeichert.

Weiters kann in zumindest einem Gelenk des Parallelogramms eine elektromagnetische Bremse angeordnet sein. Auf diese Weise kann eine komfortable Sperrung der Beweglichkeit des Parallelogramms bzw. der Befestigungseinrichtung erzielt werden.

Die vorliegende Erfindung wird anhand der beigefügten Figuren näher erläutert. Darin zeigen:
Fig. 1 ein Anwendungsbeispiel der erfindungsgemäßen Befestigungseinrichtung zur Befestigung eines Arm-Exoskeletts an einem Rollstuhl;
Fig. 2 eine Ausführungsvariante der Befestigungseinrichtung in vergrößerter Darstellung;
Fig. 3A eine Prinzipdarstellung des verstellbaren Parallelogramms der Befestigungseinrichtung in der Seitenansicht und Draufsicht in einer Position;
Fig. 3B die Prinzipskizze des verstellbaren Parallelogramms in Seitenansicht und Draufsicht in einer gegenüber Fig. 3A geänderten Position;
Fig. 4 eine Variante der Befestigungseinrichtung mit einer daran angeordneten Gewichtskompensations-Einrichtung;
Fig. 5 die Ausführungsvariante der Befestigungseinrichtung gemäß Fig. 4 mit teilweise geschnittener Darstellung der Gewichtskompensations-Einrichtung; und
Fig. 6 eine Prinzipskizze einer weiteren Ausführungsvariante einer Befestigungseinrichtung.

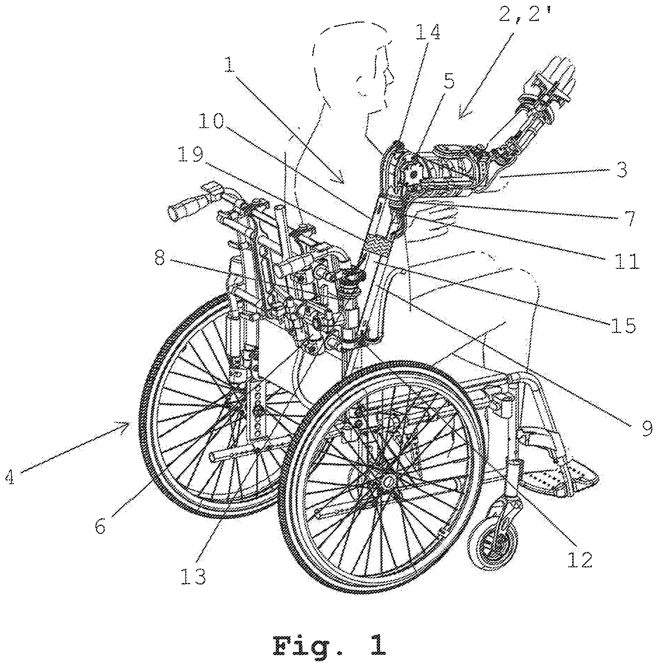

Fig. 1 zeigt eine Anwendung der erfindungsgemäßen Befestigungseinrichtung 1 zur Befestigung einer Arm-Stützstruktur 2, insbesondere eines Arm-Exoskeletts 2', an einem Rollstuhl 4. Die Befestigungseinrichtung 1 weist ein Verbindungselement 5 zur Verbindung mit der Arm-Stützstruktur 2 bzw. dem Arm-Exoskelett 2' auf, und ein Verbindungselement 6 zur Verbindung mit dem Rollstuhl 4. Anstelle eines Rollstuhls 4 kann natürlich auch ein Sessel oder ein hinter einem Sessel angeordnetes Gestell oder dgl. zur Abstützung herangezogen werden (nicht dargestellt). Herkömmliche Arm-Stützstrukturen 2 bzw. Arm-Exoskelette 2' werden üblicherweise am Körper des Patienten getragen oder starr mit dem Rollstuhl 4 oder dgl. verbunden. Beim Tragen der Arm-Stützstruktur 2 am Körper des Patienten muss dieser nachteiligerweise das Gewicht der Arm-Stützstruktur 2 tragen, was bei der Ausführung verschiedener Bewegungen des Arms 3 nachteilig sein kann. Aus diesem Grund wird das Gewicht der Arm-Stützstruktur 2 meist am Rollstuhl 4 oder dgl. abgestützt, was jedoch bei herkömmlichen Befestigungseinrichtungen zu Einschränkungen der Bewegungsfreiheit im Bereich des Oberkörpers des Patienten führt.

Die erfindungsgemäße Befestigungseinrichtung 1 erlaubt dem Patienten eine Neigung des Oberkörpers und eine Drehung des Schultergelenks jenes Armes 3, an dem die Arm-Stützstruktur 2 angeordnet ist, sodass zumindest in Pausen zwischen Bewegungstherapien eine gewisse Bewegungsfreiheit für den Patienten gegeben ist. Zu diesem Zweck ist das Verbindungselement 5 zur Verbindung mit der Arm-Stützstruktur 2 durch eine im Wesentlichen vertikal angeordnete Verbindungsstange 7 gebildet, und das Verbindungselement 6 zur Verbindung mit dem Rollstuhl 4 oder dgl. durch eine im Wesentlichen vertikal angeordnete und drehbare Verbindungsstange 8 gebildet. Die Verbindungsstangen 7, 8 werden durch zwei parallele Querstangen 9, 10 über Drehgelenke 11, 12, 13, 14 derart beweglich miteinander verbunden, sodass die Verbindungsstangen 7, 8 und die Querstangen 9, 10 ein verstellbares Parallelogramm 15 bilden. Somit ist die mit der Arm-Stützstruktur 2 verbindbare Verbindungsstange 7 sowohl in der Höhe h als auch im Winkel δ gegenüber der Verbindungsstange 8 zur Verbindung mit dem Rollstuhl 4 oder dgl. verstellbar, und erlaubt dem Patienten eine Bewegung insbesondere Neigung des Oberkörpers nach vorne und Drehung des mit der Arm-Stützstruktur 2 verbundenen Armes 3.

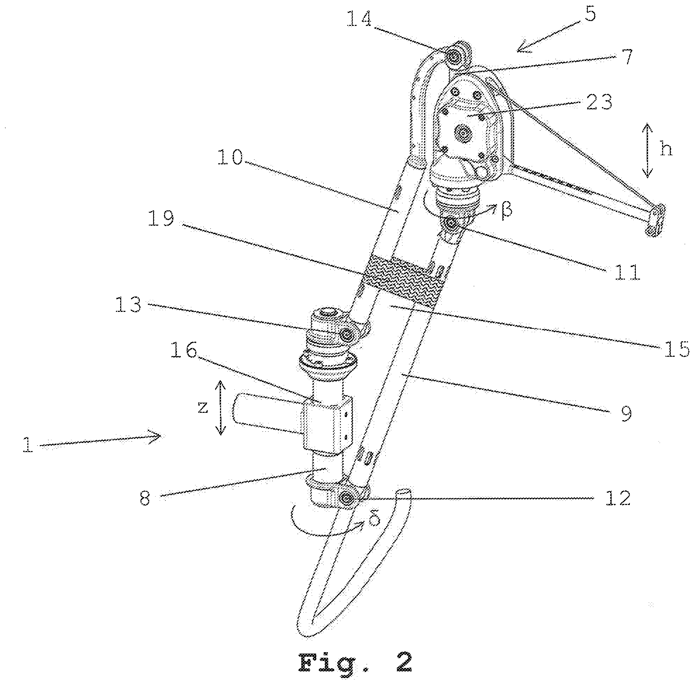

Fig. 2 zeigt eine Detailansicht einer Ausführungsform der erfindungsgemäßen Befestigungseinrichtung 1, bei der das Verbindungselement 5 zur Verbindung mit der Arm-Stützstruktur 2 (nicht dargestellt) durch eine im Wesentlichen vertikal angeordnete Verbindungsstange 7 gebildet ist und das Verbindungselement 6 zur Verbindung mit dem Rollstuhl 4 oder dgl. (nicht dargestellt) durch eine im Wesentlichen vertikal angeordnete und um einen Winkel δ drehbare Verbindungsstange 8 gebildet ist. Zwischen den Verbindungsstangen 7, 8 sind die parallelen Querstangen 9, 10 über die Gelenke 11, 12, 13, 14 beweglich miteinander verbunden und bilden ein verstellbares Parallelogramm 15. Somit kann die mit der Arm-Stützstruktur 2 verbindbare Verbindungsstange 7 sowohl in der Höhe h als auch im Winkel δ gegenüber der Verbindungsstange 8 zur Verbindung mit dem Rollstuhl 4 oder dgl. verstellt werden. Die Verbindungsstange 7 kann, wie im dargestellten Ausführungsbeispiel, auch durch einen Teil der Arm-Stützstruktur 2 bzw. des Arm-Exoskeletts 2', wie z.B. dem Schultergelenksmodul 23, gebildet sein.

Optional kann die mit dem Rollstuhl 4 oder dgl. verbindbare Verbindungsstange 8 des Parallelogramms 15 auch in Längsrichtung (Pfeil z) verschiebbar angeordnet sein, wobei die Verschiebung durch eine Einrichtung 16, wie z.B. Moosgummi, gedämpft werden kann.

Die mit der Arm-Stützstruktur 2 verbindbare Verbindungsstange 7 des Parallelogramms 15 ist drehbar um einen Winkel β angeordnet, um eine noch größere Bewegungsfreiheit für den Patienten erzielen zu können.

Die Verbindungsstangen 7, 8 und bzw. oder die Querstangen 9, 10 sind vorzugsweise aus faserverstärktem Kunststoff, insbesondere aus pultrudiertem Carbonfaserkunststoff, gebildet. Diese Werkstoffe weisen besonders hohe Festigkeit bei gleichzeitig niedrigem Gewicht auf.

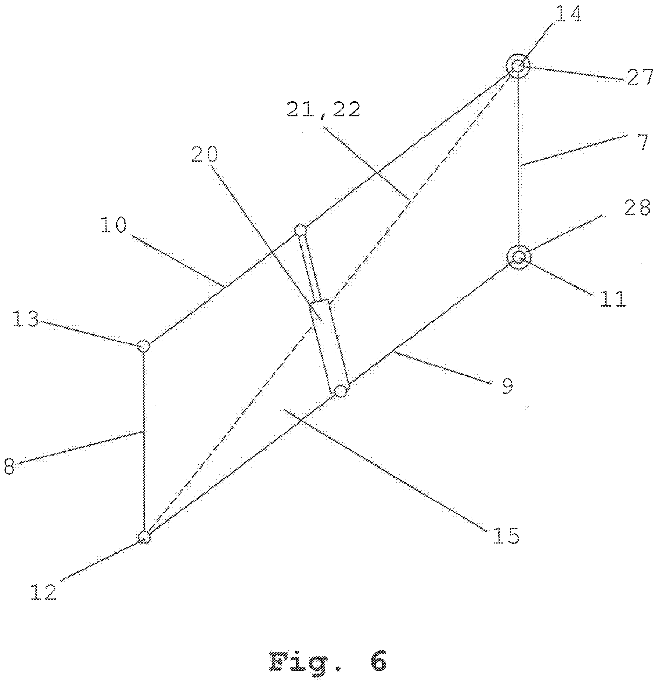

Zwischen den Querstangen 9, 10 des Parallelogramms 15 kann ein Federelement 18 angeordnet sein, welches das Parallelogramm 15 automatisch in seine Ausgangslage zurückbewegt. Das Federelement 18 kann beispielsweise durch zumindest ein elastisches Band 19 gebildet sein. Alternativ dazu kann auch eine Gasdruckfeder 20 zur Bildung des Federelements 18 eingesetzt werden (s. Fig. 6).

Wenn das Parallelogramm 15 gesperrt werden soll, um eine Bewegung zumindest zeitweise, beispielsweise während bestimmter Bewegungen des Armes 3 des Patienten, zu bewirken, kann ein entsprechendes Sperrelement 21 (s. Fig. 6) vorgesehen sein oder das Federelement 18 auch sperrbar ausgebildet werden.

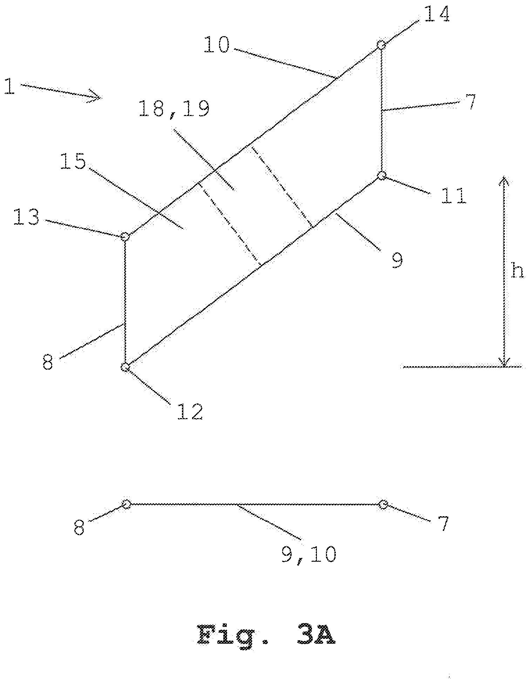

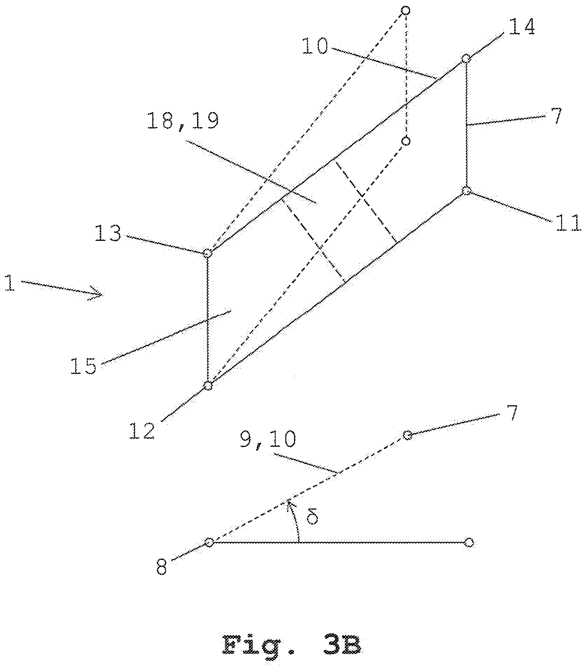

Fig. 3A und 3B zeigen eine Prinzipskizze des verstellbaren Parallelogramms 15 der Befestigungseinrichtung 1 in verschiedenen Positionen in der Seitenansicht und Draufsicht. Die Befestigungseinrichtung 1 ist in Fig. 3B gegenüber der Position gemäß Fig. 3A sowohl hinsichtlich des Winkels δ als auch hinsichtlich der Höhe h verändert worden, wodurch eine Bewegungsfreiheit des mit der Arm-Stützstruktur 2 verbundenen Patienten ermöglicht wird.

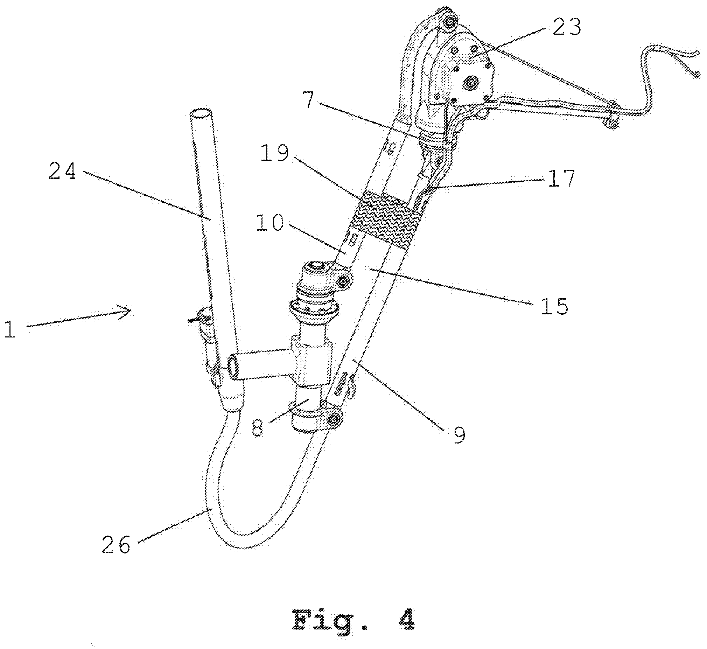

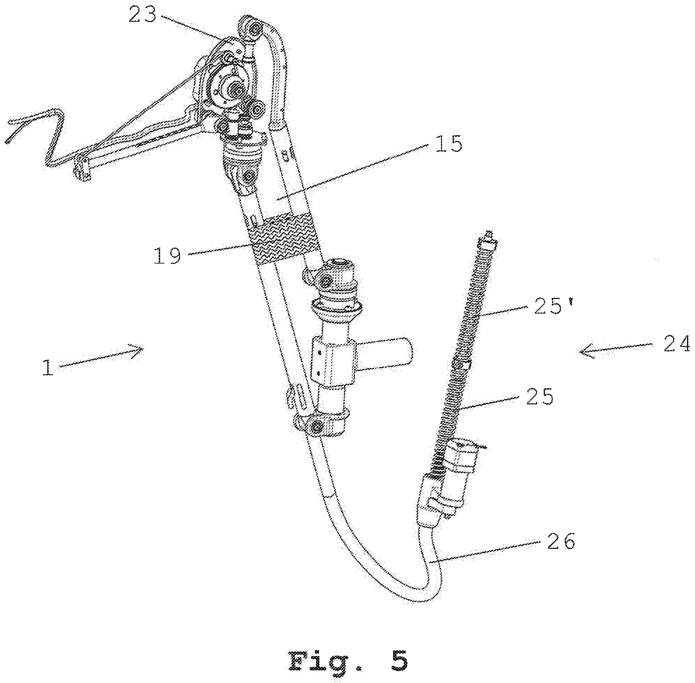

Fig. 4 zeigt eine erweiterte Ausführungsvariante der Befestigungseinrichtung 1, bei der die Verbindungsstangen 7, 8 und bzw. oder Querstangen 9, 10 zumindest teilweise hohl ausgebildet sind, sodass Leitungen 17 in den Verbindungsstangen 7, 8 bzw. Querstangen 9, 10 geführt werden können. Die Verbindungsstange 7 zur Verbindung mit der Arm-Stützstruktur 2 bzw. dem Arm-Exoskelett 2' ist zumindest teilweise durch das Schultergelenksmodul 23 des Arm-Exoskeletts 2' gebildet, welches mit einer Gewichtskompensations-Einrichtung 24 verbunden ist. Die Gewichtskompensations-Einrichtung 24 ist über einen Bowdenzug 26, der innerhalb der Querstangen 9 verläuft, mit dem Schultergelenksmodul 23 verbunden und kann aus zwei Federn 25, 25' (s. Fig. 5) bestehen, wobei eine Feder 25' zuschaltbar ausgebildet ist, sodass eine Anpassung an das zu kompensierende Gewicht möglich ist.

Schließlich zeigt Fig. 6 eine Prinzipdarstellung eines Ausführungsbeispiels der Befestigungseinrichtung 1, wobei das Federelement 18 zwischen den Querstangen 9, 10 des Parallelogramms 15 durch eine Gasdruckfeder 20 gebildet ist. Zusätzlich ist ein Sperrelement 21 durch eine diagonal zwischen den Querstangen 9, 10 angeordnete Stange 22 angedeutet. In den Gelenken 11, 12, 13, 14 des Parallelogramms 15 kann zumindest ein Winkelencoder 27 angeordnet sein, der die jeweilige Stellung des jeweiligen Gelenks 11, 12, 13, 14 an eine entsprechende Einrichtung, beispielsweise einen Computer (nicht dargestellt), weiterleitet. Weiters kann in den Gelenken 11, 12, 13, 14 eine elektromagnetische Bremse 28 angeordnet sein, um das Parallelogramm 15 elektronisch sperren zu können.

Die vorliegenden Befestigungseinrichtung 1 zeichnet sich durch einen relativ einfachen und kostengünstigen Aufbau aus und bietet einem Patienten, der mit der Arm-Stützstruktur 2 ausgestattet wird, eine Bewegungsfreiheit in den Pausen oder während der Bewegungen des Armes.

## Patentansprüche

1. Einrichtung (1) zur Befestigung einer Arm-Stützstruktur (2) zur Unterstützung der Bewegung eines Armes (3), beispielsweise einer Orthese oder eines Arm-Exoskeletts (2'), an einem Rollstuhl (4) oder dgl., mit einem Verbindungselement (5) zur Verbindung mit der Arm-Stützstruktur (2), und mit einem Verbindungselement (6) zur Verbindung mit dem Rollstuhl (4) oder dgl., wobei das Verbindungselement (5) zur Verbindung mit der Arm-Stützstruktur (2) durch eine im Wesentlichen vertikal angeordnete, um einen Winkel β drehbare Verbindungsstange (7) gebildet ist, und das Verbindungselement (6) zur Verbindung mit dem Rollstuhl (4) oder dgl. durch eine im Wesentlichen vertikal angeordnete, drehbare Verbindungsstange (8) gebildet ist, und die Verbindungsstangen (7, 8) durch zwei parallele Querstangen (9, 10) über Drehgelenke (11, 12, 13, 14) beweglich miteinander verbunden sind, **dadurch gekennzeichnet, dass** die Verbindungsstangen (7, 8) und die Querstangen (9, 10) ein verstellbares Parallelogramm (15) bilden, sodass die mit der Arm-Stützstruktur (2) verbindbare Verbindungsstange (7) sowohl in der Höhe (h), als auch in einem Winkel δ gegenüber der Verbindungsstange (8) zur Verbindung mit dem Rollstuhl (4) oder dgl. verstellbar ist.

2. Befestigungseinrichtung (1) nach Anspruch 1, **dadurch gekennzeichnet, dass** die mit dem Rollstuhl (4) oder dgl. verbindbare Verbindungsstange (8) in Längsrichtung verschiebbar angeordnet ist.

3. Befestigungseinrichtung (1) nach Anspruch 2, **dadurch gekennzeichnet, dass** eine Einrichtung (16) zur Dämpfung der Verschiebung der Verbindungsstange (8) in Längsrichtung vorgesehen ist.

4. Befestigungseinrichtung (1) nach einem der Ansprüche 1 bis 3, **dadurch gekennzeichnet, dass** die Verbindungsstangen (7, 8) und bzw. oder die Querstangen (9, 10) aus faserverstärktem Kunststoff, insbesondere aus pultrudiertem Carbon-Faser-Kunststoff, gebildet sind.

5. Befestigungseinrichtung (1) nach Anspruch 4, **dadurch gekennzeichnet, dass** die Verbindungsstangen (7, 8) und bzw. oder die Querstangen (9, 10) hohl und zur Führung von Leitungen (17) oder dgl. ausgebildet sind.

6. Befestigungseinrichtung (1) nach einem der Ansprüche 1 bis 5, **dadurch gekennzeichnet, dass** zwischen den Querstangen (9, 10) des Parallelogramms (15) ein Federelement (18) angeordnet ist.

7. Befestigungseinrichtung (1) nach Anspruch 6, **dadurch gekennzeichnet, dass** das Federelement (18) durch zumindest ein elastisches Band (19) gebildet ist.

8. Befestigungseinrichtung (1) nach Anspruch 6, **dadurch gekennzeichnet, dass** das Federelement (18) durch zumindest eine Gasdruckfeder (20) gebildet ist.

9. Befestigungseinrichtung (1) nach einem der Ansprüche 1 bis 8, **dadurch gekennzeichnet, dass** zwischen den Querstangen (9, 10) des Parallelogramms (15) ein Sperrelement (21) zum Sperren der Verstellbarkeit des Parallelogramms (15) vorgesehen ist oder das Federelement (18) sperrbar ausgebildet ist.

10. Befestigungseinrichtung (1) nach Anspruch 9, **dadurch gekennzeichnet, dass** das Sperrelement (21) durch eine diagonal zwischen den Querstangen (9, 10) angeordnete Stange (22) gebildet ist.

11. Befestigungseinrichtung (1) nach einem der Ansprüche 1 bis 10, **dadurch gekennzeichnet, dass** die Arm-Stützstruktur (2) durch ein Arm-Exoskelett (2') mit einem Schultergelenksmodul (23) gebildet ist, und die Verbindungsstange (7) zur Verbindung mit der Arm-Stützstruktur (2) zumindest teilweise durch das Schultergelenksmodul (23) des Arm-Exoskeletts (2') gebildet ist.

12. Befestigungseinrichtung (1) nach Anspruch 11, **dadurch gekennzeichnet, dass** das Schultergelenksmodul (23) mit einer Gewichtskompensations-Einrichtung (24) verbunden ist.

13. Befestigungseinrichtung (1) nach Anspruch 12, **dadurch gekennzeichnet, dass** die Gewichtskompensations-Einrichtung (24) durch zumindest eine Feder (25, 25') gebildet ist, welche über einen innerhalb der Querstangen (9, 10) verlaufenden Bowdenzug (26) verbunden ist.

14. Befestigungseinrichtung (1) nach Anspruch 13, **dadurch gekennzeichnet, dass** zumindest eine Feder (25') zuschaltbar ausgebildet ist.

15. Befestigungseinrichtung (1) nach einem der Ansprüche 1 bis 14, **dadurch gekennzeichnet, dass** in zumindest einem Gelenk (11, 12, 13, 14) des Parallelogramms (15) ein Winkelencoder (27) angeordnet ist.

16. Befestigungseinrichtung (1) nach Anspruch einem der Ansprüche 1 bis 15, **dadurch gekennzeichnet, dass** in zumindest einem Gelenk (11, 12, 13, 14) des Parallelogramms (15) eine elektromagnetische Bremse (28) angeordnet ist.

## Claims

1. Device (1) for attaching an arm support structure (2) for supporting the movement of an arm (3), for example an orthosis or an arm exoskeleton (2'), to a wheelchair (4) or the like, having a connecting element (5) for connection to the arm support structure (2), and having a connecting element (6) for connection to the wheelchair (4) or the like, the connecting element (5) for connection to the arm support structure (2) being formed by a connecting rod (7) which is arranged substantially vertically and is rotatable by an angle β, and the connecting element (6) for connection to the wheelchair (4) or the like being formed by a connecting rod (8) which is arranged substantially vertically and is rotatable, and the connecting rods (7, 8) being movably interconnected by two parallel transverse rods (9, 10) via pivot joints (11, 12, 13, 14), **characterised in that** the connecting rods (7, 8) and the transverse rods (9, 10) form an adjustable parallelogram (15), so that the connecting rod (7) that can be connected to the arm support structure (2) is adjustable both in height (h) and at an angle δ relative to the connecting rod (8) for connection to the wheelchair (4) or the like.

2. Attaching device (1) according to claim 1, **characterised in that** the connecting rod (8) which can be connected to the wheelchair (4) or the like is arranged so as to be displaceable in the longitudinal direction.

3. Attaching device (1) according to claim 2, **characterised in that** a device (16) is provided for damping the displacement of the connecting rod (8) in the longitudinal direction.

4. Attaching device (1) according to any of claims 1 to 3, **characterised in that** the connecting rods (7, 8) and/or the transverse rods (9, 10) are made from fibre-reinforced plastics material, in particular from pultruded carbon fibre plastics material.

5. Attaching device (1) according to claim 4, **characterised in that** the connecting rods (7, 8) and/or the transverse rods (9, 10) are hollow and are designed for guiding lines (17) or the like.

6. Attaching device (1) according to any of claims 1 to 5, **characterised in that** a spring element (18) is arranged between the transverse rods (9, 10) of the parallelogram (15).

7. Attaching device (1) according to claim 6, **characterised in that** the spring element (18) is formed by at least one elastic band (19).

8. Attaching device (1) according to claim 6, **characterised in that** the spring element (18) is formed by at least one gas pressure spring (20).

9. Attaching device (1) according to any of claims 1 to 8,
**characterized in that** a blocking element (21) for blocking the adjustability of the parallelogram (15) is provided between the transverse rods (9, 10) of the parallelogram (15) or the spring element (18) is designed to be blockable.

10. Attaching device (1) according to claim 9, **characterised in that** the locking element (21) is formed by a rod (22) arranged diagonally between the transverse rods (9, 10).

11. Attaching device (1) according to any of claims 1 to 10,
**characterized in that** the arm support structure (2) is formed by an arm exoskeleton (2') with a shoulder joint module (23), and the connecting rod (7) for connection to the arm support structure (2) is formed at least in part by the shoulder joint module (23) of the arm exoskeleton (2').

12. Attaching device (1) according to claim 11, **characterised in that** the shoulder joint module (23) is connected to a weight compensation device (24).

13. Attaching device (1) according to claim 12, **characterised in that** the weight compensation device (24) is formed by at least one spring (25, 25') which is connected via a Bowden cable (26) extending within the transverse rods (9, 10).

14. Attaching device (1) according to claim 13, **characterised in that** at least one spring (25') is designed so as to be able to be activated.

15. Attaching device (1) according to any of claims 1 to 14, **characterized in that** an angle encoder (27) is arranged in at least one joint (11, 12, 13, 14) of the parallelogram (15).

16. Attaching device (1) according to any of claims 1 to 15,
**characterized in that** an electro-magnetic brake (28) is arranged in at least one joint (11, 12, 13, 14) of the parallelogram (15).

## Revendications

1. Dispositif (1) pour la fixation d'une structure de soutien de bras (2) pour soutenir le mouvement d'un bras (3), par exemple d'une orthèse ou d'un exosquelette de bras (2'), sur un fauteuil roulant (4) ou similaire, avec un élément de liaison (5) pour la liaison avec la structure de soutien de bras (2), et avec un élément de liaison (6) pour la liaison avec le fauteuil roulant (4) ou similaire, dans lequel l'élément de liaison (5) pour la liaison avec la structure de soutien de bras (2) est formé par une barre de liaison (7) disposée sensiblement verticalement, pouvant tourner d'un angle β, et l'élément de liaison (6) pour la liaison avec le fauteuil roulant (4) ou similaire est formé par une barre de liaison (8) pouvant tourner, disposée sensiblement verticalement, et les barres de liaison (7, 8) sont reliées l'une à l'autre de manière mobile par deux barres transversales (9, 10) parallèles par l'intermédiaire de pivots (11, 12, 13, 14), **caractérisé en ce que** les barres de liaison (7, 8) et les barres transversales (9, 10) forment un parallélogramme réglable (15), de sorte que la barre de liaison (7) pouvant être reliée avec la structure de soutien de bras (2) est réglable tant en hauteur (h) que selon un angle δ par rapport à la barre de liaison (8) pour la liaison avec le fauteuil roulant (4) ou similaire.

2. Dispositif de fixation (1) selon la revendication 1, **caractérisé en ce que** la barre de liaison (8) pouvant être reliée au fauteuil roulant (4) ou similaire est agencée pour être déplaçable dans le sens longitudinal.

3. Dispositif de fixation (1) selon la revendication 2, **caractérisé en ce qu'**un dispositif (16) pour l'amortissement du déplacement de la barre de liaison (8) dans le sens longitudinal est prévu.

4. Dispositif de fixation (1) selon l'une des revendications 1 à 3, **caractérisé en ce que** les barres de liaison (7, 8) et/ou les barres transversales (9, 10) sont formées en matière synthétique renforcée de fibres, en particulier en matière synthétique à base de fibres de carbone pultrudée.

5. Dispositif de fixation (1) selon la revendication 4, **caractérisé en ce que** les barres de liaison (7, 8) et/ou les barres transversales (9, 10) sont agencées creuses et pour le guidage de conduites (17) ou similaires.

6. Dispositif de fixation (1) selon l'une des revendications 1 à 5, **caractérisé en ce qu'**un élément formant ressort (18) est disposé entre les barres transversales (9, 10) du parallélogramme (15).

7. Dispositif de fixation (1) selon la revendication 6, **caractérisé en ce que** l'élément formant ressort (18) est formé par au moins une bande élastique (19).

8. Dispositif de fixation (1) selon la revendication 6, **caractérisé en ce que** l'élément formant ressort (18) est formé par au moins un ressort à pression de gaz (20).

9. Dispositif de fixation (1) selon l'une des revendications 1 à 8, **caractérisé en ce qu'**entre les barres transversales (9, 10) du parallélogramme (15) un élément de verrouillage (21) pour le verrouillage de la capacité de réglage du parallélogramme (15) est prévu ou l'élément à ressort (18) est conçu pour pouvoir être verrouillé.

10. Dispositif de fixation (1) selon la revendication 9, **caractérisé en ce que** l'élément de verrouillage (21) est formé par une barre (22) disposée de manière diagonale entre les barres transversales (9, 10).

11. Dispositif de fixation (1) selon l'une des revendications 1 à 10, **caractérisé en ce que** la structure de soutien de bras (2) est formée par un exosquelette de bras (2') avec un module d'articulation de l'épaule (23), et la barre de liaison (7) pour la liaison avec la structure de soutien de bras (2) est formée au moins partiellement par le module d'articulation de l'épaule (23) de l'exosquelette de bras (2').

12. Dispositif de fixation (1) selon la revendication 11, **caractérisé en ce que** le module d'articulation de l'épaule (23) est relié à un dispositif de compensation de poids (24).

13. Dispositif de fixation (1) selon la revendication 12, **caractérisé en ce que** le dispositif de compensation de poids (24) est formé par au moins un ressort (25, 25') qui est relié par l'intermédiaire d'un câble Bowden (26) s'étendant à l'intérieur des barres transversales (9, 10).

14. Dispositif de fixation (1) selon la revendication 13, **caractérisé en ce qu'**au moins un ressort (25') est conçu pour pouvoir être déclenché.

15. Dispositif de fixation (1) selon l'une des revendications 1 à 14, **caractérisé en ce qu'**un codeur angulaire (27) est disposé dans au moins une articulation (11, 12, 13, 14) du parallélogramme (15).

16. Dispositif de fixation (1) selon l'une des revendications 1 à 15, **caractérisé en ce qu'**un frein électromagnétique (28) est disposé dans au moins une articulation (11, 12, 13, 14) du parallélogramme (15).
